Europäisches Patentamt

European Patent Office

Office européen des brevets

(19)

(11) Publication number: **0 185 517**
**B1**

(12) **EUROPEAN PATENT SPECIFICATION**

(45) Date of publication of the patent specification:
20.06.90

(21) Application number: **85309048.8**

(22) Date of filing: **12.12.85**

(51) Int. Cl.⁵: **A61F 5/44**

(54) **Fluid collection device.**

(30) Priority: **13.12.84 GB 8431418**
**12.10.85 GB 8525207**

(43) Date of publication of application:
**25.06.86 Bulletin 86/26**

(45) Publication of the grant of the patent:
**20.06.90 Bulletin 90/25**

(84) Designated Contracting States:
**BE CH DE FR GB IT LI NL SE**

(56) References cited:
**EP-A- 0 109 217**
**DE-A- 2 540 612**
**DE-B- 2 009 161**
**US-A- 3 349 768**

(73) Proprietor: **Thomas, Madeline, Gastons Farm Plaistow Road Chiddingfold, Godalming Surrey GU8 4SX(GB)**
Proprietor: **Thomas, Stephen John, Gastons Farm Plaistow Road Chiddingfold, Godalming Surrey GU8 4SX(GB)**

(72) Inventor: **Thomas, Madeline, Gastons Farm Plaistow Road Chiddingfold, Godalming Surrey GU8 4SX(GB)**

(74) Representative: **Selby-Lowndes, Guy Francis Charles, Moonrakers Durfold Wood, Plaistow Billinghurst West Sussex RH14 0PL(GB)**

ACTORUM AG

## Description

This invention relates to devices for the collection of fluids such as urine.

The care of elderly and disabled female patients frequently centres around the problems associated with urination. Where the patients are bed-ridden or confined to a wheel chair they are frequently catheterised as it seldom possible to provide at short notice the necessary assistance whenever they need to urinate. In many cases the patients are able to sit up in bed but unable to cope with the traditional slipper bed pan on their own. Catheterisation for long periods is unpleasant for the patient and may lead to infection or irritation and generally lowers the quality of life.

Various devices have been proposed for collecting or directing the flow of urine from females. These may be broadly classified into so-called incontinence devices which are designed to be worn continously by patients who are usually mobile and urination aids for use with patients who are continent but disabled or immobile. The latter class of devices can frequently be used by the patients themselves when needed.

GB-A-1 422 638 describes a urinary aid intended for use by disabled females. This device is complex as it is designed to hold the labia open during use. This arrangement is unnecessary and frequently inconvenient for the user.

US-A-3 349 768 describes a suction evacuated urinal which is shaped to provide full tissue support for the external genitalia when positioned in contact therewith. The urinal is intended for patients in hospital and requires a vacuum supply for its use.

None of the urinary aids which have been proposed so far are satisfactory for use by a female patient who wishes to urinate at will and without assistance but is confined to a bed or a wheelchair. They require a degree of manual dexterity, both hands to use them, and are to some extent invasive which is frequently disagreeable for the patient.

The present invention provides a urine collecting device for use by females comprising a smooth edged upper portion (1), adapted to enclose the vulva, and a lower reservoir portion (4) having at its base a drainage pipe (6, 16) for connection to a drainage tube, characterised in that the reservoir portion (4, 15) has a fluid capacity from 200 to 700 ml and has a substantially flat base (10, 20) and the drainage pipe (6, 16) initially has a direction of flow parallel to the base.

The drainage pipe is connected by a flexible pipe to a urine receiving vessel. The receiving vessel may be a flexible polymer bag such as those regularly used by catheterised patients. Bags of this type are described in UK patent specification 2153236. Preferably the receiving vessel is a hollow collecting vessel such as a bottle.

In a preferred form of the invention the drainage pipe is connected to a flexible pipe during manufacture to ensure a fluid tight seal. The nature of the connection to the flexible pipe is dependant upon the materials chosen for the two components. An adequate fluid tight seal will usually be obtained if the flexible pipe is pushed over the drainage pipe while the former is in a state of enhanced flexibility due to heat or immersion in a low boiling point solvent. The length of flexible pipe will depend upon the situation in which the device is used. In general it is believed that a length of 750 mm is more than adequate for most purposes and any surplus can be cut off before connection to the collection vessel. The flexible pipe may be fabricated from a polyvinyl ester, polyolefin or similar material. It is most important that the material can withstand some sterilisation treatment, chemical or thermal, during the lifetime of the associated device.

The smooth edged upper portion of the device preferably comprises sheet material inwardly curved towards a central aperture. In this configuration the urine discharged by the patient enters through the central aperture and the underside of the edge catches splashed fluid and reduces the possibility of spillage after use. While it is possible to construct the upper portion of the device from a suitable metal it is preferably, for reasons of comfort, fabricated from a material with low thermal conductivity, such as a synthetic polymer, so that it is warm to the touch.

The lower reservoir portion receives and retains the discharged urine until it has flowed through the drainage pipe. The rate at which urine is discharged from a patient may often be greater than the rate at which it can flow away so that the device must be able to hold an appreciable volume of fluid. In general a capacity of about 200 ml is adequate for the embodiment of the device used by seated patients but up to 600ml or more is necessary in the embodiment used by prone patients. The capacity is dependant upon the calibre of the drainage pipe and, as flow is normally under the action of gravity, the vertical distance between the device and the collecting vessel.

In order that the device can lie flat on a supporting surface, e.g. a bed or the seat of a chair, when in use the drainage pipe should project from the side of the base of the lower reservoir portion. Preferably the drainage pipe projects from the front of the device when in use. The outer end of the pipe may be smooth, ribbed, tapered or otherwise adapted to allow the flexible drainage tube to be connected. The urine flows through the flexible drainage pipe into the collecting vessel. Flexible polymeric tubing is commonly used having an internal diameter of 8mm to 10mm. Urine collecting bags are well known and widely used by the nursing profession. A wide range of such bags is available both calibrated and uncalibrated. In the preferred embodiment the collecting vessel is a rigid or semi-rigid bottle. For reasons of cost and weight bottles fabricated, for instance by the blow-moulding technique, of synthetic polymers such as olefins or polyesters are convenient. Alternatives such as disposable papier mache containers can be used.

Due to the comparatively small bore of the tubing used to carry urine to the collecting bags in common use air-locks can occur which may impede the flow of the fluid. In one preferred form of the device air relief means are used to allow the air to escape from the tube and attached collection vessel. The air-relief means comprises a small tube which leads from the drainage pipe to the upper portion with its exit below the curved surface. Urine collection bags are available which include an air relief valve. In the case of bottles a small air vent at the top is sufficient. There are many well known techniques for assisting fluid flow using air-relief valves and tubes which are suitable.

In a further embodiment the collecting vessel has associated suction means to draw the fluid from the device into the vessel. Such suction means can comprise a powered pump or a partial vacuum in the collecting vessel.

The choice of materials for fabricating devices according to the invention is dependant upon the life expectancy of the device and the method of manufacture chosen. If the device is to be regularly reused the materials from which it is made must be capable of withstanding chemical and/or thermal sterilisation treatments. Disposable or short life devices may be made from materials such as papier mache or polystyrene foam. In general most thermoplastic synthetic polymers are suitable such as polystyrene, polyolefins, polyvinyl esters, polycarbonates, cellulose esters, acrylic polymers and copolymers such as ABS. Other materials such as thermosetting resins may be used in appropriate circumstances alone or reinforced.

The method by which the device is manufactured will depend upon the material chosen for it. In a preferred method of manufacture the device is made from two mating components comprising the upper portion and the lower portion. Each portion may be made by the well-known vacuum forming technique from a thermoplastic sheet of material such as polystyrene sheet. After the drainage pipe has been fitted to the lower portion the two components are mated and a fluid tight seal is formed by use of an adhesive or ultra-sonic welding. Such sealing processes are well known.

Where manufacture of different fluid capacity devices is taking place it is convenient to design the upper portion so that it mates with two or more alternative lower reservoir portions, e.g. one with a capacity of 200 ml and another of 600 ml.

The drainage pipe will usually be made from thicker material than the upper and lower portions of the device. For this reason it is preferably fabricated as a separate operation and added to the lower portion. The pipe must form a fluid tight seal with the reservoir. Such seals are easily made using adhesives or cold setting putties.

The invention will now be described with reference to the accompanying figures in which:

Figure 1 is a plan view of a device according to the invention,

Figure 2 is a side view of the device,

Figure 3 is a cross section across the device along the line a-a in Figure 2,

Figure 4 is a plan view of an alternative embodiment of a device according to the invention, and

Figure 5 is a cross section of the device along the line b-b in Figure 4.

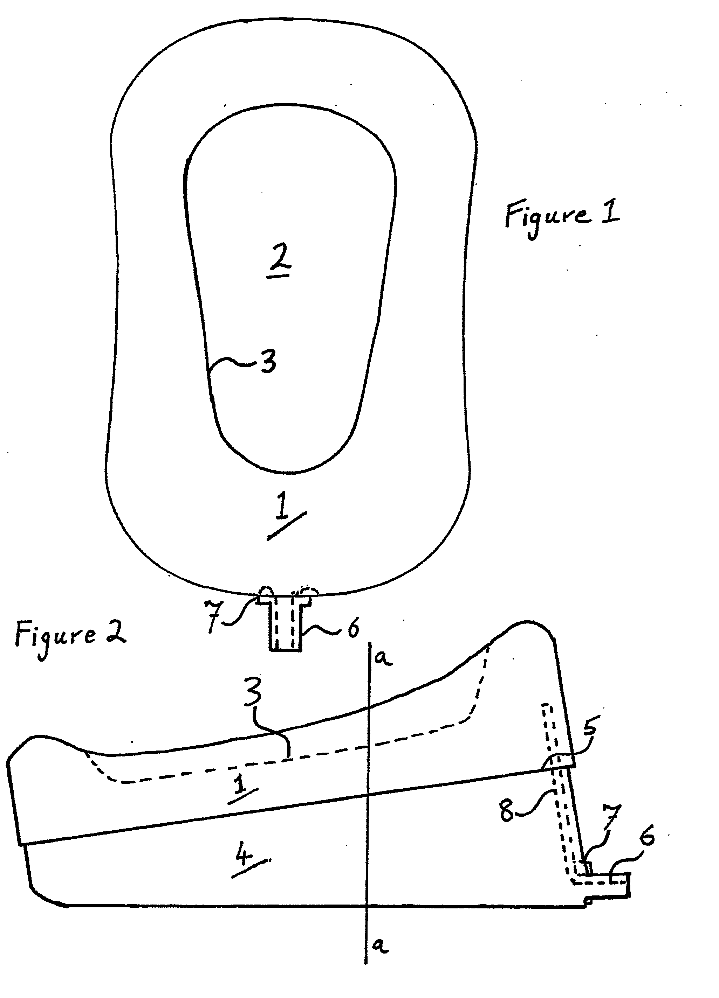

The top surface 1 of the smooth edged upper portion of the urine collecting device, see Figure 1, has a central aperture 2 to allow entry of urine. The edge 3 of the aperture 2 is preferably at least 10 mm lower than the upper edge of the top surface 1 to minimise splashing and spilling.

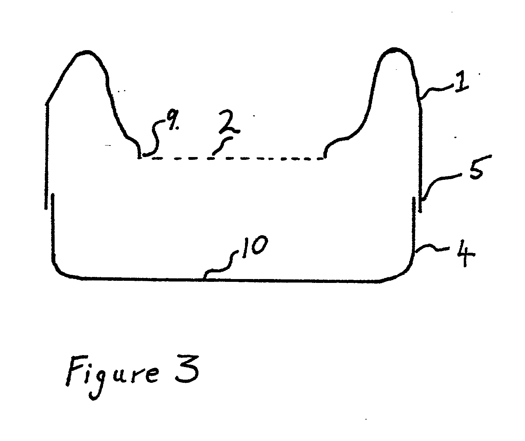

The nature of the top surface, 1, of the upper portion may be more clearly seen with reference to Figure 3. The top surface, 1, has re-entrant edges, 9, which ensure that splashes are retained and return to the base 10.

The lower reservoir portion, see Figure 2, comprises a fluid-tight container, 4, which mates with the upper portion, 1. The two portions may be fabricated separately and joined by a fluid tight seal, 5, as shown in Figures 2 and 3, or be fabricated as an integral unit. At its base the reservoir portion has a drainage pipe, 6, attached by a fluid-tight seal, 7.

The bore of the drainage pipe, 6, should be as large as possible to facilitate the flow of fluid out of the reservoir portion. The outer dimension of the drainage pipe, 6, is chosen to form a fluid-tight fit with the flexible piping, not shown, commonly used to transfer urine into collecting vessels.

In one embodiment the drainage pipe, 6, contains a smaller tube, 8, which acts as a vent and allows air to escape from the urine collecting vessel when the fluid flows under the action of gravity. Alternatively the collecting vessel may contain its own air relief valve or vent to allow the escape of air displaced by the fluid entering it.

In use the patient usually confined to a bed or a wheel chair presses the top surface 1 against the pubic area so that the vulva is enclosed and faces the aperture 2. The rate of flow of urine released by the user is frequently greater than the maximum rate of flow through the pipe 6 under the action of gravity. For this reason an accumulation of urine occurs in the reservoir portion 4. After release of urine ceases the user may tip the device so that all the fluid contained in it is directed into the pipe 6. Where the user is prone the capacity of the container 4 is about 600 ml. For seated users or users capable of sitting up in bed a capacity of 200 ml is usually sufficient.

Flow through the pipe 6 to the fluid collection vessel not shown may be under the action of gravity or assisted by the action of a pump or a partial vacuum.

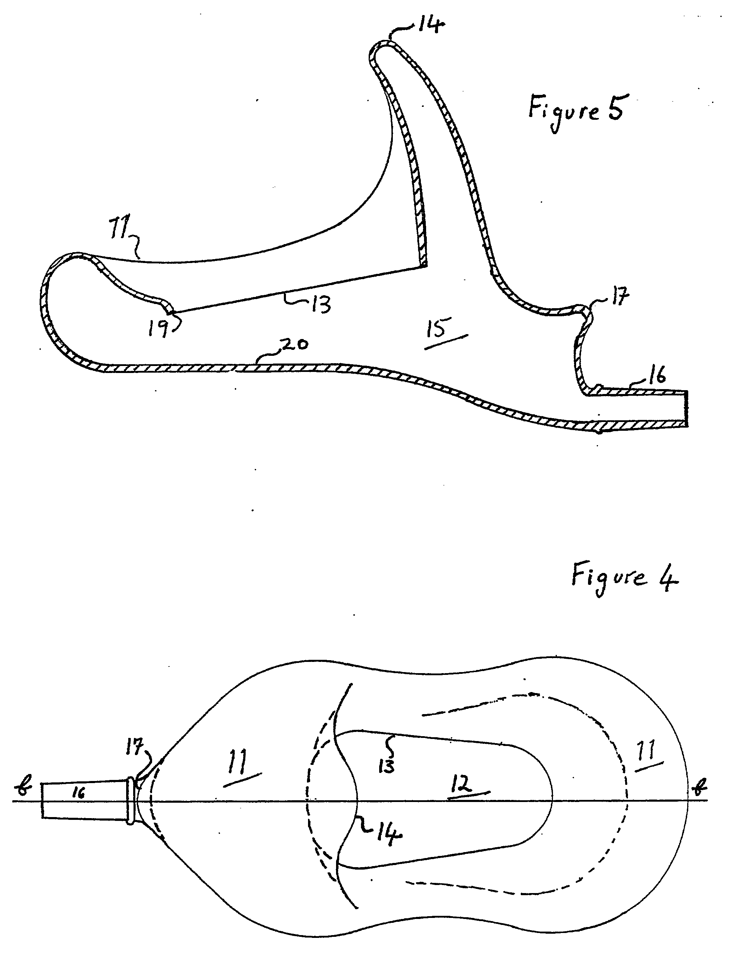

The second embodiment is particularly adapted for use with bedridden patients who may be seated or supine, see Figures 4 and 5.

The top surface 11 of the smooth edged upper portion of the urine collecting device see Figure 4 has a central aperture 12 to allow entry of urine. The edge 13 of the aperture 12 is preferably at least 10 mm lower than the upper edge of the top surface 11 to minimise splashing and spilling. A raised rim 14 protruding upwards at the front of the aperture 12 assists the user to locate the device by acting as a stop. The rim 14 preferably extends from 30mm to 80mm above the top surface 11. The rim 14 also acts as a shield to prevent spillage when the device contains fluid and is tipped forward.

The lower reservoir portion 15, see Figure 5, has at its base a drainage pipe 16 which is shown as part of the integral unit but which may be a separate pipe attached by a fluid-tight seal. A protrusion 17 to assist handling extends outwardly from the front of the device above the drainage tube 16.

The nature of the top surface 11 of the upper portion may be more clearly seen with reference to Figure 5. The top surface 11 has re-entrant edge 9, which are directed to the base 20 to ensure that splashes are retained within the reservoir portion 15. In this embodiment the upper portion and the lower reservoir portion are shown fabricated as an integral unit however they could be formed using two mating components joined by a fluid-tight seal as described with respect to the first embodiment.

As described with respect to the first embodiment the bore of the drainage pipe 16 should be as large as possible to facilitate the flow of fluid out of the reservoir portion. The outer dimension of the drainage pipe 16, is chosen to form a fluid-tight fit with the flexible piping not shown commonly used to transfer urine into collecting vessels.

In use the patient usually confined to a bed or a wheel chair presses the top surface 11 against the pubic area so that the vulva is enclosed and faces the aperture 12. To facilitate the correct location of the device the lip 14 is held against the pubic bone. This facility is most useful for users such as paraplegics who have little or no sensitivity in this region. By sliding the device forward the user can feel resistance when the rim 14 meets the pubic bone and know that the correct position has been reached.

A further aid to handling the device is provided by the protrusion 17. The protrusion 17 engages the intersection between the thumb and forefinger and allows the users hand io guide the device into the correct position. The protrusion prevents the hand slipping upwards when sliding the device under the user's body even when it is fabricated from smooth material.

In an alternative embodiment, not illustrated, instead of the protrusion 17 the front of the device may carry a handle. The handle may be an integral part of the front of the device or fabricated separately and fixed to the body of the device by means of adhesives, welding or any of the other well-known bonding techniques.

The base 20 is substantially flat but may have a short sloping portion, see Figure 5. The drainage pipe 16 however has a direction of flow parallel to the flat portion of the base 20.

The under surface of the base 20 may be patterned or otherwise treated to increase friction against the surface on which it is placed when in use. It is found that a ribbed base prevents the device slipping when in contact with a bed sheet so that the user need not hold the device in position when using it.

## Claims

1. A urine collecting device for use by females comprising a smooth edged upper portion (1), adapted to enclose the vulva, and a lower reservoir portion (4) having at its base a drainage pipe (6, 16) for connection to a drainage tube, characterised in that the reservoir portion (4, 15) has a fluid capacity from 200 to 700 ml and has a flat base (10, 20) and the drainage pipe (6, 16) initially has a direction of flow parallel to the base.

2. The device according to claim 1, characterised in that the smooth edged upper portion (1) has edges (9, 10) forming a central aperture (2, 12) and that the edges (9, 19) are directed to the base of the lower reservoir portion (4) to minimise splashing or spilling.

3. The device according to either claim 1 or claim 2, characterised in that the drainage pipe includes venting means.

4. The device according to claim 1 characterised, in that it is constructed from a synthetic polymer.

5. The device according to any of the preceding claims, characterised in that the container comprises two mating components (1, 4).

6. The device according to claim 5, characterised in that the lower component is a fluid-tight container having the drainage pipe (6, 16) at its base.

7. The device according to claims 5 or 6, characterised in that the two components are joined together by a fluid-tight seal (5).

8. The device according to claims 1 to 4, characterised in that the upper and lower portions form an integral unit (15).

9. The device according to any of the preceding claims, characterised in that the drainage pipe carries a flexible drainage tube.

10. The device according to any of the preceding claims, characterised in that the smooth edged upper portion has a raised rim protruding upwards at its front (14).

11. The device according to claim 12, characterised in that the rim protrudes from 30mm to 80mm above the upper surface of the upper portion.

12. The device according to any of the preceding claims, characterised in that a protrusion (17) to assist handling extends outwardly from the front of the device above the drainage pipe.

13. The device according to any of the preceding claims 1 to 11, characterised in that the front of the device carries a handle.

## Patentansprüche

1. Eine Harnsammelvorrichtung zum Gebrauch durch Frauen, mit einem glattrandigen oberen Teil (1), der so beschaffen ist, daß er die Vulva umschließt, und einem unteren Reservoirteil (4) mit einem Ablaufrohr (6, 16) zum Anschluß an einen Ablaufschlauch an seinem Boden, dadurch gekennzeichnet, daß der Reservoirteil (4, 15) ein Flüssigkeitsfassungsvermögen von 200 bis 700 ml und einen flachen Boden (10, 20) hat und daß die Fließrichtung des Ablaufrohrs (6, 16) anfänglich zu dem Boden parallel ist.

2. Die Vorrichtung nach Anspruch 1, dadurch gekennzeichnet, daß der glattrandige obere Teil (1) eine mittlere Öffnung (2, 12) bildende Ränder (9, 10) aufweist und daß die Ränder (9, 19) auf den Boden des unteren Reservoirteils (4) zuweisen, um Verspritzen bzw. Verschütten auf ein Mindestmaß einzuschränken.

3. Die Vorrichtung nach Anspruch 1 oder Anspruch 2, dadurch gekennzeichnet, daß das Ablaufrohr ein Entlüftungsmittel umfaßt.

4. Die Vorrichtung nach Anspruch 1, dadurch gekennzeichnet, daß sie aus einem synthetischen Polymer gefertigt ist.

5. Die Vorrichtung nach einem der vorstehenden Ansprüche, dadurch gekennzeichnet, daß der Behälter zwei passende Teile (1, 4) umfaßt.

6. Die Vorrichtung nach Anspruch 5, dadurch gekennzeichnet, daß der untere Teil ein flüssigkeitsdichter Behälter mit einem Ablaufrohr (6, 16) an seinem Boden ist.

7. Die Vorrichtung nach Anspruch 5 oder 6, dadurch gekennzeichnet, daß die beiden Teile durch eine flüssigkeitsdichte Dichtung (5) miteinander verbunden sind.

8. Die Vorrichtung nach den Ansprüchen 1 bis 4, dadurch gekennzeichnet, daß die oberen und unteren Teile eine integrale Einheit (15) bilden.

9. Die Vorrichtung nach einem der vorstehenden Ansprüche, dadurch gekennzeichnet, daß das Ablaufrohr mit einem Ablaufschlauch versehen wird.

10. Die Vorrichtung nach einem der vorstehenden Ansprüche, dadurch gekennzeichnet, daß der glattrandige obere Teil einen erhöhten Rand aufweist, der an der Vorderseite (14) hochsteht.

11. Die Vorrichtung nach Anspruch 12, dadurch gekennzeichnet, daß der Rand 30 bis 80 mm oberhalb der oberen Fläche des oberen Teils hochsteht.

12. Die Vorrichtung nach einem der vorstehenden Ansprüche, dadurch gekennzeichnet, daß sich ein Vorsprung (17) als Handhabungshilfe von der Vorderseite der Vorrichtung oberhalb des Ablaufrohrs nach außen erstreckt.

13. Die Vorrichtung nach einem der vorstehenden Ansprüche 1 bis 11, dadurch gekennzeichnet, daß die Vorderseite der Vorrichtung mit einem Griff versehen ist.

## Revendications

1. Dispositif pour la collecte de l'urine destiné à un usage féminin comprenant une partie supérieure à rebord lisse (1), adaptée pour entourer la vulve, et une partie inférieure faisant fonction de réservoir (4) comportant en sa base un conduit d'évacuation (6, 16) devant s'emboîter sur un tuyau de drainage, caractérisé en ce que la partie-réservoir (4, 15) offre une capacité en fluide de 200 à 700 ml et comporte une base plate (10, 20) et le conduit d'évacuation (6, 16) offre un sens d'écoulement initial parallèle à la base.

2. Dispositif selon la revendication 1, caractérisé en ce que la partie supérieure à rebord lisse (1) comporte des bords (9, 10) formant une ouverture centrale (2, 12) et que les bords (9, 19) sont tournés vers la base de la partie-réservoir inférieure (4) afin de minimiser les éclaboussures ou fuites.

3. Dispositif selon la revendication 1 ou la revendication 2, caractérisé en ce que le conduit d'évacuation comporte des moyens d'aération.

4. Dispositif selon la revendication 1, caractérisé en ce qu'il est fabriqué en un polymère synthétique.

5. Dispositif selon l'une des revendications précédentes, caractérisé en ce que le conteneur se compose de deux éléments emboîtés l'un dans l'autre (1, 4).

6. Dispositif selon la revendication 5, caractérisé en ce que l'élément inférieur est un réservoire étanche aux fluides comportant en sa base un conduit d'évacuation (6, 16).

7. Dispositif selon la revendication 5 ou 6, caractérisé en ce que les deux éléments sont assemblés par un joint étanche aux fluides (5).

8. Dispositif selon les revendications 1 à 4, caractérisé en ce que les parties supérieure et inférieure forment une unité intégrale (15).

9. Dispositif selon l'une des revendications précédentes, caractérisé en ce que le conduit d'évacuation comporte un tuyau flexible d'évacuation.

10. Dispositif selon l'une des revendications précédentes, caractérisé en ce que la partie supérieure à rebord lisse comporte un bord surélevé en sa partie avant (14).

11. Dispositif selon la revendication 12, caractérisé en ce que le bord surélevé fait saillie de 30mm à 80mm au-dessus de la surface supérieure de la partie supérieure.

12. Dispositif selon l'une des revendications précédentes, caractérisé en ce qu'une partie fait saillie (17), pour faciliter la manutention, vers l'extérieur à partir de l'avant du dispositif au-dessus du conduit d'évacuation.

13. Dispositif selon l'une des revendications précédentes 1 à 11, caractérisé en ce que l'avant du dispositif comporte une poignée.

EP 0 185 517 B1

Figure 1

2

3

1

Figure 2

7        6

3            a

5

1        8

4            7

6

a

Figure 3

EP 0 185 517 B1

Figure 5

Figure 4